Europäisches Patentamt

European Patent Office

Office européen des brevets

(11) Veröffentlichungsnummer: **0 360 139**
**A2**

(12) **EUROPÄISCHE PATENTANMELDUNG**

(21) Anmeldenummer: 89116920.3

(51) Int. Cl.⁵: **A61B 17/58**

(22) Anmeldetag: 13.09.89

(30) Priorität: 17.09.88 DE 3831657

(43) Veröffentlichungstag der Anmeldung:
28.03.90 Patentblatt 90/13

(84) Benannte Vertragsstaaten:
**BE CH DE ES FR GB GR IT LI LU NL SE**

(71) Anmelder: **BOEHRINGER INGELHEIM KG**

**D-6507 Ingelheim am Rhein(DE)**
(84) **BE CH DE ES FR GR IT LI LU NL SE**

Anmelder: **BOEHRINGER INGELHEIM
INTERNATIONAL G.M.B.H.**

**D-6507 Ingelheim am Rhein(DE)**
(84) **GB**

(72) Erfinder: **Eitenmüller, Jürgen, Dr.
Römerstrasse 27
D-5024 Brauweiler(DE)**
Erfinder: **Offergeld, Heinz
Ebelshof 4
D-4050 Mönchengladbach 1(DE)**
Erfinder: **Michaeli, Walther, Prof. Dr.
Nizzaallee 30
D-5100 Aachen(DE)**

(54) **Vorrichtung zur Osteosynthese und Verfahren zu ihrer Herstellung.**

(57) Vorrichtung zur Osteosynthese und Verfahren zu
ihrer Herstellung.

EP 0 360 139 A2

## Vorrichtung zur Osteosynthese und Verfahren zu ihrer Herstellung

Die Erfindung betrifft eine resorbierbare Vorrichtung zur Osteosynthese bestehend aus einer Osteosyntheseplatte und einer Befestigungsvorrichtung sowie Verfahren zu ihrer Herstellung.

Die Anwendung der stabilen Plattenosteosynthese, mit deren Hilfe eine mechanische Verbindung zwischen den beiden Bruchenden einer Fraktur erreicht wird, ist an sich bekannt und kann zur Stabilisierung der verschiedensten Frakturen am menschlichen Skelett nutzbar gemacht werden. Herkömmlicherweise werden die zur Osteosynthese benötigten Platten, Schrauben, Nägel oder Nadeln aus Metall - Edelstählen oder Chrom-Nickel-Legierungen (z.B. vom Typ L 316) -hergestellt, womit im wesentlichen zwei gravierende Nachteile verbunden sind: Zum einen ist der Steifheitsgrad der Metallimplantate deutlich höher als derjenige menschlicher Knochen. Die hieraus resultierende "stress protection" hat einen Knochenabbau (Osteoporosie) zur Folge, welcher seinerseits das Risiko einer erneuten Fraktur - nach dem Entfernen der Metallplatte - erhöht. Zum anderen muß das Implantat nach der Beendigung des Heilungsprozesses in einer weiteren Operation entfernt werden, was nicht nur zu einer mit weiteren Risiken verbundenen Belastung des Patienten führt, sondern auch mit erheblichen Heilungskosten verbunden ist. Weiterhin läßt die ansteigende Zahl der auftretenden Chrom-Nickel-Allergien und die damit verbundenen Komplikationen den Einsatz derartiger Materialien problematisch erscheinen.

Diese Nachteile können durch den Einsatz sogenannter bio-resorbierbarer Polymere überwunden werden. Als biologisch resorbierbare Polymere werden im allgemeinen und im Sinne der Erfindung solche Polymere verstanden, die unter physiologischen Bedingungen zu körpereigenen Substanzen abgebaut und mit dem Stoffwechselkreislauf ausgeschieden werden. Die Verwendung bio-resorbierbarer Materialien bei der Plattenosteosynthese bietet den Vorteil, daß die sonst notwendige Zweitoperation zur Entnahme des Implantates entfällt. Der resorptionsbedingte Abbau des Implantates, mit dem eine entsprechende Minderung der mechanischen Stabilität verbunden ist und der eine zunehmende funktionelle Belastung des Knochens bedingt, welche ihrerseits eine weitere funktionelle Strukturierung im Bruchspalt ermöglicht, verhindert auf diesem Wege die unerwünschte Auswirkung der "stress protection".

Zahlreiche Patente bzw. Patentanmeldungen haben resorbierbare Implantate für die Plattenosteosynthese zum Gegenstand:
EP 0258 692, US-PS 46 55 777, EP 0204 931, EP 0011 528 und US-PS 43 29 743.

In diesen Patenten wird davon ausgegangen, daß die Festigkeit der resorbierbaren Polymere für einen Einsatz in der Osteosynthese nicht ausreicht. Daher wurde vorgeschlagen, verschiedenartige Fasern als Verstärkungsmaterial zur Erhöhung der mechanischen Belastbarkeit einzusetzen, wobei diese aus resorbierbaren oder in der Regel aber aus nicht-resorbierbaren Materialien bestehen können. Die Form der Platten oder Schrauben wird dabei fast vollständig von den entsprechenden Osteosynthesevorrichtungen aus Metall übernommen. Es hat sich in der Praxis jedoch gezeigt, daß die für die "Metallosteosynthese" bewährten Formen und Konstruktionen bei der Verwendung von resorbierbaren Polymeren entscheidende Nachteile aufweisen. So wurde bislang beispielsweise außer acht gelassen, daß die aus der Verwendung von Kunststoff resultierenden Gestaltungsmöglichkeiten andere, wesentlich belastungsgerechtere Konstruktionen zulassen, deren Verwendung zudem für den operierenden Arzt mit erheblichen Erleichterungen verbunden sein kann.
Herkömmliche Osteosyntheseplatten aus resorbierbaren Polymeren weisen beispielsweise den Nachteil auf, daß sie - insbesondere im Bereich der Schraubenlöcher -relativ leicht brechen.

Die bisher im Stand der Technik vorgeschlagenen Ausführungsformen für Schrauben weisen ebenfalls Nachteile auf, so zum Beispiel, daß sehr viele verschiedene Längen zur Verfügung stehen müssen, um den Anforderungen jeder individuellen Fraktur gerecht werden zu können. Die Länge der benötigten Schrauben wird zur Zeit noch mit Meßfühlern an den in den betreffenden Knochen vorgebohrten Löchern ermittelt. Dies setzt bei dem jeweiligen Operateur ein großes Maß an Erfahrung voraus. Entsprechend der gemessenen Länge können dann erst während der Operation geeignete Schrauben ausgesucht werden. Bei der Verwendung von Metallschrauben erweist sich ferner der Umstand als nachteilig, daß beim Festziehen der Schrauben das Gewinde im Knochen überdreht werden kann, woraus sich entweder die Notwendigkeit ergibt, ein Ersatzloch bohren zu müssen, oder aber eine neue Schraube mit einem größerem Durchmesser verwendet werden muß, was jedoch erst nach einer aufwendigen Entfernung der ursprünglich eingedrehten Schraube möglich ist. Auf der anderen Seite erwächst beim Einsatz von Schrauben aus resorbierbaren Materialien der Nachteil, daß beim Einschrauben zwar nicht das Gewinde im Knochen zerstört wird, sondern - aufgrund der geringen mechanischen Festigkeit - das Gewinde derSchraube selbst oder aber die Schraube unterhalb des Schraubenkopfes abreißt. Diese Schrauben müssen zum Entfernen ausgebohrt und anschließend ersetzt werden.

Diese vergleichsweise geringe mechanische Stabilität bedingt, daß die Schrauben aus resorbierbaren Polymeren zum Befestigen der Platte nicht so fest angezogen werden können, daß die erforderliche geringe Verschiebebeweglichkeit zwischen der Platte und den Knochenhälften erzielt wird und somit ein einwandfreies Zusammenwachsen der Bruchstücke nicht gewährleistet ist. Je geringer die freie Beweglichkeit der Bruchstücke (Verschiebebeweglichkeit) ist, desto größer sind die Heilungsaussichten des Bruches.

Ein weiterer Nachteil von resorbierbaren Syntheseschrauben besteht darin, daß sie keinen Röntgenkontrast erzeugen und somit der Operateur keine Möglichkeit besitzt, die richtige Lage der Schrauben zu beurteilen.

Es ist die Aufgabe der vorliegenden Erfindung, eine resorbierbare Vorrichtung zur Osteosynthese zur Verfügung zu stellen, die die Verschiebebeweglichkeit zwischen der Platte und den Knochenbruchstücken auf das für das komplikationslose Zusammenwachsen der Knochen erforderliche Maß reduziert.

Es ist weiterhin die Aufgabe der vorliegenden Erfindung, Vorrichtungen zur Befestigung der Osteosyntheseplatte aus resorbierbaren Polymeren zur Verfügung zu stellen, die im Falle ihrer Beschädigung oder Zerstörung leicht aus dem Knochen entfernt und ersetzt werden können, um die eventuell notwendige Entfernung der ganzen Befestigungsvorrichtung zu vermeiden.

Eine weitere Aufgabe der vorliegenden Erfindung besteht darin, Befestigungsvorrichtungen zur Verfügung zu stellen, die hinsichtlich ihrer Länge ohne weiteren Aufwand in das jeweilige Bohrloch eingepaßt werden können, um somit eine Lagerhaltung von einer Vielzahl von Schrauben bzw. Befestigungsvorrichtungen verzichtbar zu machen.

Eine weitere Aufgabe der vorliegenden Erfindung besteht darin, eine kunststoffgerechte Gestaltung der Befestigungsvorrichtung vorzunehmen, die gegenüber der reinen Kopie der Gestalt von Metallvorlagen aus Kunststoff höhere Anzugskräfte erlaubt.

Eine weitere Aufgabe der vorliegenden Erfindung besteht darin, eine Osteosynthese-Vorrichtung aus resorbierbaren Polymeren röntgenologisch sichtbar zu machen.

Die Aufgabe der vorliegenden Erfindung wird durch eine resorbierbare Osteosynthesevorrichtung bestehend aus einer mit Schraubenlöchern versehenen Platte aus einem resorbierbaren Polymer oder Copolymer, die auf der dem Knochen zugewandten Seite mit Calciumphosphat- und/oder Hydroxylapatitgranulat beschichtet ist sowie mindestens zweier Befestigungsvorrichtungen bestehend aus einer Gewindestange und der dazugehörigen Mutter gelöst.

Geeignete resorbierbare Polymere aus denen die Platte, die Gewindestangen und die Muttern bestehen können sind Polymere und Copolymere auf der Basis folgender Monomere: L-Lactid, D,L-Lactid, Glycolid. Falls es gewünscht oder erforderlich ist, können die Polymere bzw. Copolymere durch resorbierbare Fasern verstärkt werden. Die Verfahren zur Herstellung und Bearbeitung geeigneter Polymere und Copolymere sind aus dem Stand der Technik bekannt und brauchen nicht näher erörtert werden.

Die Form der erfindungsgemäßen Platte kann in sehr weiten Bereichen beliebig variiert werden, soweit dies nicht im Widerspruch zu den Eigenschaften der verwendeten Polymermaterialien (Bruchfestigkeit, Abbaurate etc.) und der beabsichtigten Verwendung (Art des Bruches etc.) steht. Ein wesentliches Merkmal der erfindungsgemäßen Platte ist, daß sie an ihrer Unterseite - d.h. der dem Knochen zugewandten Seite -eine Beschichtung aus Calciumphosphat und/oder Hydroxylapatit - vorzugsweise in Form eines Granulates oder kleiner Kügelchen - aufweist.

Einerseits erfährt die Unterseite der erfindungsgemäßen Platte durch die Beschichtung eine Aufrauhung, die im Vergleich zu den bisher verwandten Osteosyntheseplatten aus bio-resorbierbaren Materialien bei gleicher Andruckkraft eine geringere Verschiebebeweglichkeit gewährleistet - andererseits sichert die mit Calciumphosphat bzw. Hydroxylapatit beschichtete Platte ein baldiges Anwachsen an der Knochenoberfläche, da auch industriell hergestelltes Calciumphosphatkeramik-Granulat weitestgehend dem im Knochen befindlichen Mineral entspricht.

Durch das Anwachsen der mit Calciumphosphatkeramik-Granulat beschichteten bio-resorbierbaren Osteosyntheseplatte an der Knochenoberfläche kommt es zu einer zusätzlichen mechanischen Stabilisierung der Platte auf der Knochenoberfläche, wodurch gleichzeitig die unerwünschte Beweglichkeit zwischen Knochen und Platte bei vorgegebener axialer Schraubenzugkraft auf ein Mindestmaß verringert wird. Diese kann von ausschlaggebender Bedeutung sein, falls es zu einem vorzeitigen Bruch der Befestigungsvorrichtung (nachfolgend auch als Gewindestange bezeichnet oder auch Schraube genannt) infolge beginnender Resorption und der damit verbundenen mechanischen Schwächung der Schrauben kommt, wobei davon ausgegangen werden kann, daß sich die Resorption bei den unter hoher mechanischer Belastung stehenden filigranen Schrauben schneller auswirkt als bei den wesentlich massiver ausgeführten Platten.

Durch die Calciumphosphat- bzw.

Hydroxylapatit-Beschichtung - im folgenden auch Calciumphosphatkeramik-Granulat genannt - wird zudem die Möglichkeit eröffnet, das Implantat röntgenologisch sichtbar zu machen. Auf diesem Wege können Form und Größe des Implantates einschließlich der Lagebeziehung zum stabilisierten Knochen im Röntgenbild beurteilt werden. Weiterhin ist ein eventueller Bruch oder eine spätere Dislokation der Platte im Röntgenbild zu erkennen. Die Calciumphosphatkeramiken unterliegen ebenfalls der Resorption, auch wenn diese etwas größere Zeiträume beansprucht als dies z.B. beim Polylactid der Fall ist.

Zur Herstellung der erfindungsgemäßen Platte können folgende Techniken angewandt werden: Das Calciumphosphatkeramik-Granulat wird heiß in die Oberfläche einer vorgefertigten Platte eingepresst. Die Anwendung dieser Methode hat eine mechanisch sehr feste Bindung des Granulates an dem Träger zur Folge. Die Aufbringung kann beispielsweise aber auch in der Weise erfolgen, daß z.B. eine 0,5 - 1,0 mm dicke Polylactidfolie auf eine heiße dicht liegende Granulatschicht heiß aufgepresst wird, womit ein Verbund des Granulates erzielt wird. Es erweist sich als vorteilhaft, wenn die Granulatkörner nicht zu dicht nebeneinander liegen; hierdurch wird vermieden, daß das Granulat beim Biegen der Platten abplatzt. Die so erhaltene granulatbeschichtete Polylactidfolie wird in einem geeigneten Spritzgußwerkzeug ausgelegt und mit Polylactid überspritzt, wobei es zu einer innigen Verbindung der mit Hydroxylapatit bzw. mit Calciumphosphat beschichteten Polylactidfolie mit dem durch Spritzguß erzeugten Implantatkörper kommt. Die Herstellung der granulatbeschichteten Folie kann großflächig erfolgen. Im anschließenden Schritt kann die Folie auf die gewünschte Plattengröße zugeschnitten werden.

In einer weiteren Ausführungsform können die Calciumphosphatkeramik-Granulatkörner elektrostatisch an einer Wand des Werkzeuges fixiert werden und anschließend wie oben beschrieben überspritzt werden. Weiterhin besteht die Möglichkeit die Beschichtung auf die Weise durchzuführen, daß die Granulatkörner an kleinen Bohrungen eines Spritzgußwerkzeuges, die einen kleineren Durchmesser als die Granulatkörner aufweisen, während des anschließenden Spritzvorganges durch Ansaugen in dieser Posititon gehalten werden. Die erfindungsgemäßen Platten können unter Erwärmung verformt werden, so daß sie der Knochenoberfläche angepaßt werden können. Dies kann auch vom Operateur -z.B. mit einem speziellen Heißluftgebläse oder einem für diese Zwecke speziell ausgestalteten Mikrowellengenerator - im Operationssaal durchgeführt werden. Ein nennenswertes Abplatzen der Granulat-Beschichtung ist dabei nicht zu beobachten. Die spontane maximale Zugkraft einer bei den Versuchen verwendeten Polylactid-Platte von 2000 N (ermittelt mit einer Zwick Material-Prüfmaschine) bestand nach der Hitzebehandlung der Oberfläche unverändert weiter.

In einer weiteren Ausführungsform können alle nicht dem Knochen zugewandten Flächen der Platte einschließlich die der Schraubenköpfe mit einem niedermolekularen lediglich geringe mechanische Festigkeit aufweisenden Film eines geeigneten Polymeren - z.B. mit einem Polylactidfilm - überzogen werden, welcher kolloidal verteiltes Silber enthält. Die Oberflächenbeschichtung - von der Dicke bis zu 1 mm - verhindert einerseits aufgrund der oligodynamischen antibakteriellen Wirksamkeit des Silbers eine Keimbesiedlung der Oberfläche des Implantates in den ersten Wochen nach der Operation; zum zweiten wird eine schemenhafte röntgenologische Sichtbarmachung des Implantates ermöglicht. Um eine primäre Gewebetoxizität zu vermeiden, ist es erforderlich, die der Platte näher liegenden Schichten stärker mit Silber anzureichern als diejenigen Schichten an der Oberfläche. Die höherangereicherten aber tieferliegenden Schichten, werden keinen toxikologisch bedenklichen Einfluß ausüben, da im Verlaufe der Resorption Anteile des Silbers aus den tiefergelegenen Schichten herausgelöst werden, so daß nach dem Aberodieren der jeweils an der Oberfläche liegenden Schichten das Gewebe mit einer Schicht in Kontakt kommt, die eine bereits reduzierte Konzentration an kolloidalem Silber - bezogen auf die Ausgangskonzentration - aufweist, wodurch Schädigungen des Gewebes vermieden werden.

In einer weiteren Ausführungsform kann die Calciumphosphatkeramik-Granulatschicht der Platte selbst mit feinkörnigem Silberphosphat bis zu 30 % (bezogen auf das Gesamtgewicht des Granulates) angereichert werden, um dadurch auch auf dieser Seite des Implantats eine antibakterielle Wirkung zu erzielen.

An Stelle von kolloidal verteiltem Silber können die beschriebenen Schichten auch PVP-Jod [Polyvinylpyrrolidon-Jod-Komplex bzw. Poly(1-vinyl-2-pyrrolidin-2-on)-Jod-Komplex] in Konzentrationen von 5 % bis 40 % enthalten, wodurch ebenfalls eine antibakterielle Wirkung als auch eine röntgenologische Sichtbarmachung der Platte erreicht wird.

Eine antibakterielle bzw. bakteriostatische Wirkung kann weiterhin durch Beaufschlagung mit thermostabilen Antibiotika aus verschiedenen Gruppierungen unter Bevorzugung von denjenigen Antibiotika mit breitem Wirkungsspektrum - wie z.B. Aminoglycoside, Gyrase-Hemmer oder Vancomycin - erfolgen. Bei einer derartigen Beschichtung braucht auf eine Systemtoxizität keine Rücksicht genommen werden, da nur geringe Mengen im Serum nachweisbar werden, die weit unterhalb der Toxizitätsgrenze liegen, welche bei einer intravenösen Applikation beachtet werden muß.

Die Platte selbst wird, wie aus der Entwicklung von Metallplatten zur Stabilisierung von Knochen bekannt ist, im wesentlichen durch Biegung und Zugbeanspruchung belastet. Es ist daher anzustreben, im Spritzgußverfahren durch die Anordnung der Spritzdüsen eine Längsorientierung der Molekülketten zu erreichen. Die notwendigen technischen Voraussetzungen zur Verarbeitung der Polymere sind dem Fachmann bekannt. Es wird jedoch darauf hingewiesen, daß das zu spritzende Gut extrem trocken gehalten werden muß, da bei höheren Temperaturen mit jedem Promille Wassergehalt der Anteil an Kettenabbauprodukten steigt. Dies wirkt sich direkt auf das Abbauverhalten des Polymeren aus. Wie schon erwähnt, besteht die Möglichkeit, in Pulverform vorliegende und thermostabile Antibiotika, wie z.B. Aminoglykoside oder Gyrase-Hemmer, zuzufügen. Da deren thermische Belastung beim Aufpressen nur für eine äußerst kurze Zeitspanne akut wird, kann dabei die vom jeweiligen Hersteller angegebene tolerierbare Temperaturobergrenze überschritten werden, ohne daß daraus schwerwiegende Nachteile für das Präparat wie auch für das Implantat resultieren.

Die Temperaturführung und das Nachspritzen während des Aushärtens ist derart durchzuführen, daß im Zentrum keine Hohlräume entstehen und die Wärme möglichst schnell abgeführt werden kann, um die thermische Belastung des Polymers so gering wie möglich zu halten.

In einer besonderen Ausführungsform wird die erfindungsgemäße Platte durch ein in das Polymer eingelagertes Fasermaterial verstärkt.

Als Fasermaterial wird bevorzugt verstrecktes Poly-L-lactid verwendet, während die Matrix vorzugsweise aus Poly-D,L-lactid besteht. Die Zugfestigkeit der erfindungsgemäßen Platten dieser Ausführungsform ist im Vergleich zu der nicht verstärkten Platte bis um einen Faktor von 10 größer. Vorteilhaft erweisen sich extrudierte Fasern, die beim Aushärten zusätzlich verstreckt werden. Da die Platten im wesentlichen einer Zug- und Biegebelastung ausgesetzt sind, sollten die Fasern längsorientiert sein und der Winkel, den die Fasern zueinander aufweisen, 30° nicht überschreiten. Als Temperaturschutz bei der Herstellung für die Fasern erweist sich gegebenenfalls ein dünner Überzug aus niedermolekularem L-Lactid oder D,L-Lactid von Vorteil. Auch der verstärkten Platte kann kolloidal verteiltes Silber, PVP-Jod oder ein wärmebeständiges und in Pulverform vorliegendes Antibiotikum zugemischt werden.

Die Formgebung der Platten richtet sich in erster Linie nach dem Indikationsgebiet. Trotz unterschiedlicher Länge bzw. der generell unterschiedlichen Größe der Platten - in Abhängigkeit von der geplanten Anwendung -weisen die Osteosyntheseplatten und die Befestigungsvorrichtungen vorzugsweise folgende Grundformen auf:

Fig. 1a zeigt eine bevorzugte Ausführungsform der erfindungsgemäßen Osteosyntheseplatte (1.0).

Fig. 1b zeigt einen Querschnitt durch die erfindungsgemäße Osteosyntheseplatte (1.0) aus Fig. 1a längs der Schnittlinie cc.

Fig. 1c zeigt einen Querschnitt durch die erfindungsgemäße Osteosyntheseplatte (1.0) aus Fig. 1a längs der Schnittlinie dd.

Fig. 2 zeigt eine Seitenansicht der erfindungsgemäßen Osteosyntheseplatte, die in der Mitte eine um ca. 25 % stärkere Dicke aufweist als an den Enden ($h^1$ = 1/4 $h^2$).

Fig. 3 zeigt die Gewindestange (3.0) der erfindungsgemäßen Befestigungsvorrichtung, welche ein Querloch (3.1), ein einen Röntgenkontrast erzeugendes Teilchen (3.2) und eine Verjüngung (3.3) (Sollbruchstelle) aufweist.

Fig. 4 zeigt eine bevorzugte Ausführungsform einer Mutter (4.0) der erfindungsgemäßen Befestigungsvorrichtung, welche gegebenenfalls mehrere Vorrichtungen (4.1) zur Übertragung eines Drehmomentes aufweist.

Fig. 5 zeigt eine erfindungsgemäße Osteosyntheseplatte (5.0), welche mit den erfindungsgemäßen Befestigungsvorrichtungen, Gewindestange (3.0) und der Mutter (4.0) auf den Knochenfragmenten angebracht ist.

Fig. 6 zeigt die erfindungsgemäße Vorrichtung zur Osteosynthese nach dem Abschneiden der Gewindestangen.

Fig. 7a zeigt eine weitere bevorzugte Ausführungsform einer erfindungsgemäßen Osteosyntheseplatte (7.0), welche im zentralen Teil mit in parallel zur Längsrichtung verlaufenden Verstärkungen (7.1) versehen ist sowie Verbreiterungen im Bereich der Schraubenlöcher (7.3) aufweist. Die erfindungsgemäße Osteosyntheseplatte (7.0) weist an beiden Enden Einkerbungen (7.2) zum Einsetzen eines Plattenspanngerätes auf.

Fig. 7b zeigt einen Querschnitt durch die erfindungsgemäße Osteosyntheseplatte (7.0) aus Fig. 7a längs der Schnittlinie ee.

Fig. 7c zeigt einen Querschnitt bzw. die resultierenden Querschnittsflächen bei einem Schnitt durch die erfindungsgemäße Osteosyntheseplatte (7.0) aus Fig. 7a längs der Schnittlinie ff.

Fig. 7d zeigt die Seitenansicht der erfindungsgemäßen Osteosyntheseplatte (7.0) aus Fig. 7a mit der Verstärkung (7.1) und den Einkerbungen (7.2) zum Einsetzen eines Plattenspanngerätes.

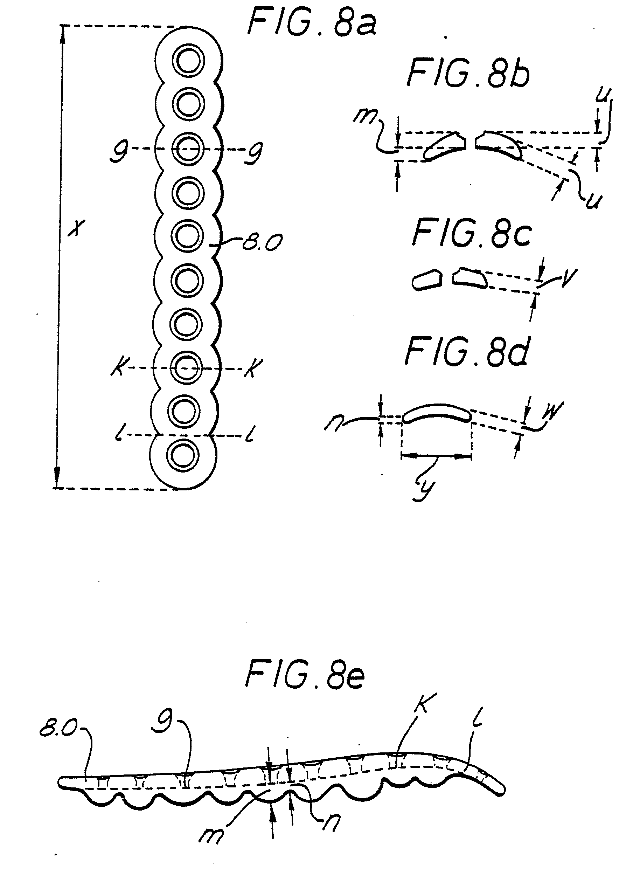

Fig. 8a zeigt eine weitere bevorzugte Ausführungsform der erfindungsgemäßen Vorrichtung zur Osteosynthese in Form einer Außenknöchelplatte (8.0) mit den Schnittlinien gg, kk und 11, wobei die Länge x der Platte etwa 10 cm beträgt.

Fig. 8b zeigt einen Querschnitt durch die erfindungsgemäße Osteosyntheseplatte (8.0) aus Fig. 8a längs der Schnittlinie gg, wobei die Dicke u der Platte ca. 2 mm und die Höhe der Wölbung m ca. 2 mm beträgt.

Fig. 8c zeigt einen Querschnitt durch die erfindungsgemäße Osteosyntheseplatte (8.0) aus Fig. 8a längs der Schnittlinie kk, wobei die Dicke der Platte v ca. 1,8 mm beträgt.

Fig. 8d zeigt einen Querschnitt durch die erfindungsgemäße Osteosyntheseplatte (8.0) aus Fig. 8a längs der Schnittlinie 11, wobei die Dicke w der Platte ca. 1,5 mm und die Höhe n der Wölbung ca. 1 mm beträgt.

Fig. 8e zeigt die Seitenansicht der Außenknöchelplatte (8.0) mit den Lagen der Schnittebenen in Fig. 8a und den Höhen der Wölbungen m und n.

Je nach Verwendungszweck bzw. Einsatzgebiet können andere Plattenabmessungen bzw. Wölbungshöhen vorteilhafter sein.

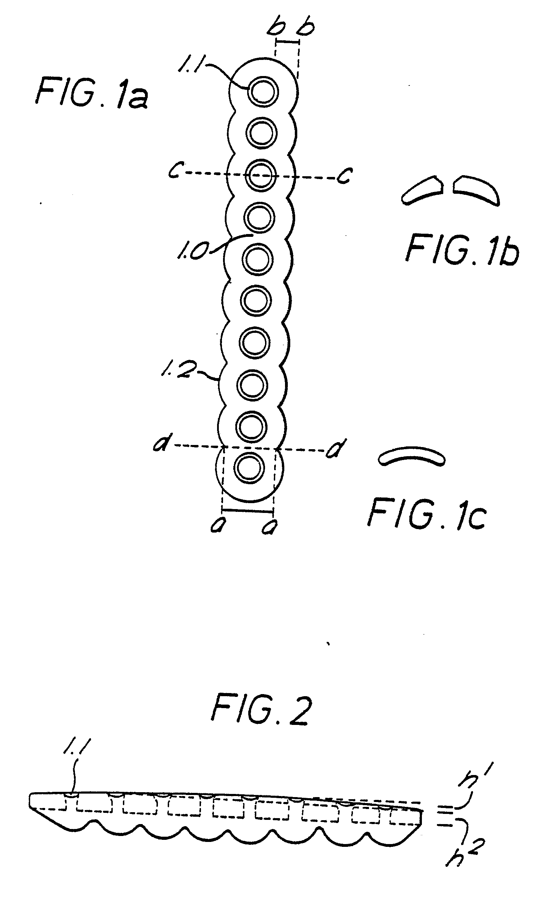

Die Platte (1.0) (Fig. 1) ist derart gestaltet, daß der Querschnitt längs der Schnittlinie bb, das heißt die Querschnitte, die längs der Schnittlinie cc, die durch den Mittelpunkt der Schraubenlöcher bzw. Befestigungslöcher (1.1) gelegt werden kann, ungefähr dem halben Querschnitt längs der Schnittlinie dd in der Lage aa der Platte (1.0) zwischen den Befestigungslöchern (1.1) entsprechen. Durch diese Formgebung wird vermieden, daß die Platte (1.0) im Bereich der Befestigungslöcher (1.1) infolge des Abbaus des Polymermaterials durch Resorption vorzeitig "geschwächt" wird. Dadurch bedingt ergibt sich eine längliche Grundform der Osteosyntheseplatte, die im Bereich der Befestigungslöcher Ausbuchtungen (1.2)

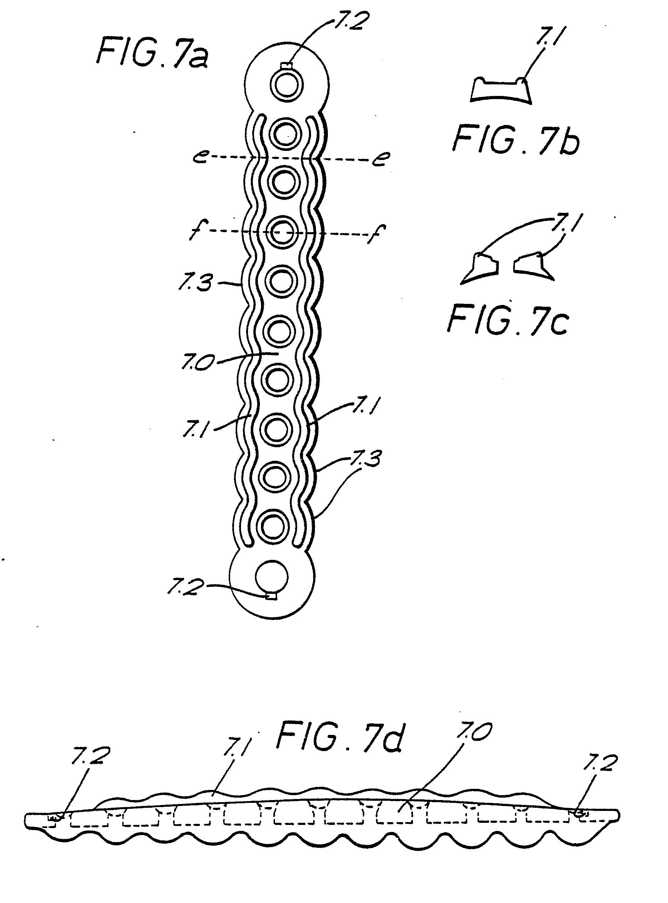

Weiterhin soll die Platte in der Mitte (Fig. 2) eine um 25 % stärkere Dicke aufweisen als an den Enden. Darüber hinaus wird durch seitliche und in Längsrichtung verlaufende Verstärkungen bzw. Verdickungen der Platte (7.1 in Fig. 7a-d) im wesentlichen im zentralen Teil der Platte eine Stabilisierung der Platte erreicht.

Dabei sollten diese Verstärkungen bzw. Verdickungen nur so hoch sein, daß sie den höchsten Punkt der Schraube nicht überragen, um keine Gesamtverdickung des Systems Platte/Schraube hervorzurufen. Die Befestigungslocheinsenkungen sind vorzugsweise sphärisch gestaltet (Fig. 1b), wobei sich gegebenenfalls Langlöcher mit schräger Anlaufbahn als vorteilhaft erweisen können.

Die erfindungsgemäße Osteosyntheseplatte wird mittels einer Befestigungsvorrichtung mit dem Knochen verbunden.

Als Befestigungsvorrichtungen sind im allgemeinen Schrauben, Gewindestäbe, Muttern oder ähnlich gestaltete Befestigungsvorrichtungen gleicher Funktion geeignet, die aus einem im Körper resorbierbarem Polymeren, wie z.B. Poly-L-lactid, Poly-D,L-lactid, Polyglycolid, Copolymeren oder Polymeren bzw. Copolymeren mit resorbierbaren Fasern bestehen.

Bei der Herstellung der Schrauben hat es sich bewährt, diese mit einem kunststoffgerechten 3-Grad-30 Grad-Sägezahngewinde zu versehen. Dieses Gewinde besitzt den Vorteil, daß die Gewindezüge des Schraubenkerns die gleiche Stärke gegenüber den Gewindezügen im Knochen und gegenüber der Schraubenmutter aufweisen. Die Kanten des Gewindes sind stumpf gehalten, um ein vorzeitiges Nachlassen der Haltekraft, wie sie bei spitzen Gewindekanten, welche die meisten Gewinde aufweisen - durch die Resorption bedingt - beobachtet werden kann, zu vermeiden. Das 3-Grad-30-Grad-Sägezahngewinde besitzt ein geringes Rückstellmoment, welches eine Lockerung der Schrauben bei den im menschlichen bzw. tierischen Körper ununterbrochen stattfindenden Mikrobewegungen verhindert, da sich die sehr weit ausgeladenden Gewindezüge breit am umgebenden Knochen abstützen können.

Generell ist eine Verbesserung der Materialeigenschaften der Schrauben durch die Anwendung von Spritzgußverfahren - wie es bei der Herstellung von Faserverbundwerkstoffen angewandt wird - möglich. Hierbei muß sowohl bei der Orientierung der Molekülketten im Schraubenkern, als auch bei der Orientierung der Fasern bei der Herstellung des Verbundmaterials eine schraubenförmige 45°-Drehung im rechtsdrehenden Sinne erzielt werden, damit die Schraube sowohl der axial längsgerichteten Schraubenzugkraft als auch der zugleich zu tolerierenden Drehkraft Stand zu halten vermag. Eine prinzipielle Materialverbesserung wird durch Anwendung des Spritzgußverfahrens (dichtere Packung des Materials sowie Quervernetzung der Molekülketten) erzielt.

Beim Beibehalten konventioneller Schrauben, welche zur Erzielung einer axialen Schraubenzugkraft unter Anwendung eines geeignet großen Drehmomentes eingedreht werden, bieten sich beispielsweise folgende Kopfformen an:

Ein bis in Ansatznähe des Schraubenkerns und damit durch den gesamten Schraubenkopf durchreichender Innensechskant, welcher - in flacherer Ausführung - von einem Außensechskant umgeben wird. In dem zirkular umlaufenden Wall des Schraubenkopfes sind weitere Einsenkungen eingearbeitet, die eine weitere Übertragung des Drehmoments ermöglichen.

Bei einer derartigen Form des Schraubenkopfes und der hierzu korrespondierenden Form des Schraubenziehers ist einerseits eine sichere Führung der Schraube möglich (die Schraube sitzt auf dem Schraubenzieher und kann auch in nach unten gerichteten Bohrungen eingeführt werden). Mit dieser Ausführungsform ist ferner eine maximale Übertragung des Drehmomentes gewährleistet, ohne dabei Gefahr zu laufen, daß es bei dem Versuch, das Drehmoment vom Schraubenzieher auf die Schraube zu übertragen zu einem Abdrehen der Kanten kommt. Solche Schraubenköpfe lassen sich im Spritzgußverfahren problemlos in großer Zahl und zu niedrigen Kosten herstellen.

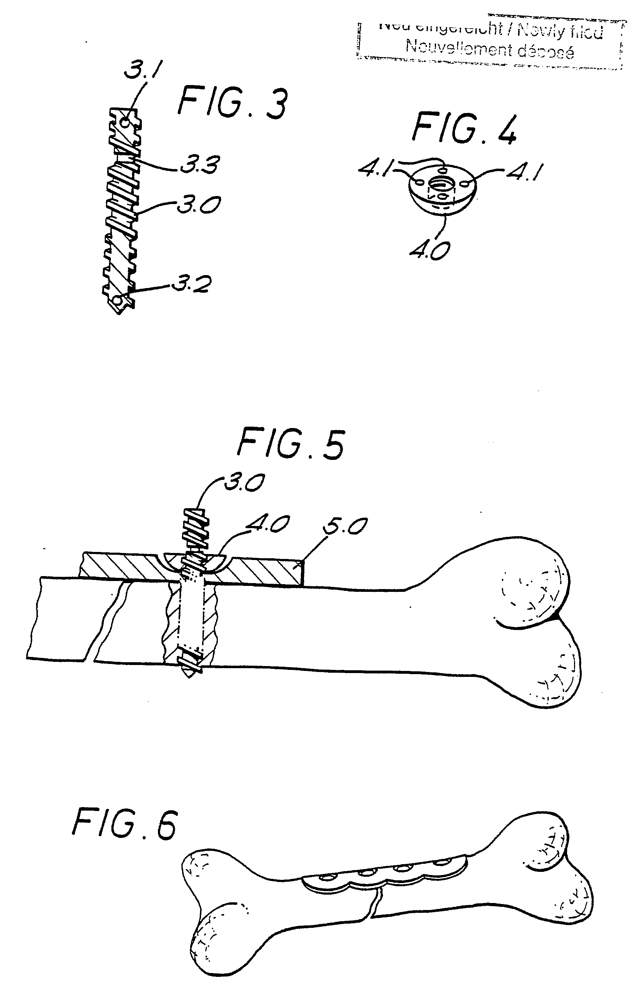

Die erfindungsgemäße resorbierbare Befestigungsvorrichtung besteht aus einer Gewindestange (3.0) (Fig. 3) und einer dazugehörigen Mutter (4.0) mit Innengewinde (Fig. 4), wobei die Gewindestange an einem Ende die Möglichkeit eines Werkzeugansatzes zur Drehmomentübertragung bietet (z.B. 3.1 in Fig. 3).

Dieser Ansatz kann das Gewinde selbst sein -vorzugsweise jedoch als Loch, Schlitz oder Innenbzw. Außen-Sechskant - ausgestaltet sein. Die Befestigungseinheit kann mit jedem entsprechend geeigneten Werkzeug, dessen Anzugskraft regelbar sein sollte, in den Knochen eingeschraubt und festgezogen werden. Die Gewindestange (3.0) kann in einer bevorzugten Ausführungsform über eine Sollbruchstelle (3.3) (Fig. 3) verfügen, die bei einem Versagen des Gewindestabes beim Eindrehen ein müheloses Erneuern der Befestigungseinheit ermöglicht.

Das untere Ende der Gewindestange (3.0) (Fig. 3), kann - wie an späterer Stelle beschrieben wird - mit einem Röntgenschatten erzeugenden Material bzw. Teilchen (3.2) (Fig. 3) versehen sein, das sich in fein verteilter Schicht auf der Oberfläche oder in kompakter Form innerhalb oder lediglich nur an oder in der Spitze der Schraube bzw. des Gewindestabes befindet (Fig. 3). Das Röntgenschatten erzeugende Material kann selbst bio-resorbierbarer oder auch nicht bio-resorbierbarer Natur sein.

Zur Befestigung der erfindungsgemäßen Platte bieten sich - je nach Ausgestaltung der Gewindestange - bzw. des Gewindestabes verschiedene Vorgehensweisen an.

a) Der Gewindestab enthält keine besonderen Ausgestaltungen zum Ansatz eines Werkzeuges (d.h. einer Vorrichtung zum Eindrehen des Gewindestabes). In diesem Fall ist eine speziell gestaltete Vorrichtung zum Einschrauben notwendig.

Auf dem oberen Ende des Gewindestabes ist eine Mutter aufgeschraubt, welche einen Sechskant aufweist und die am unteren Ende sphärisch zusammenläuft. Die Mutter wird mit dem daran befindlichen Gewindestab in die Sechskant-Aufnehmung einer speziellen Vorrichtung eingesteckt und das Gewinde durch die eingesteckte Mutter hindurch in das Innengewinde in der Vorrichtung eingeschraubt.

In dieser Position kann durch Drehen der Rändelmutter an der Vorrichtung der Gewindestab in den Knochen eingedreht werden, worauf der Gewindestab bis zum Anschlag fingerfest angezogen wird. Anschließend wird über einen Spannmechanismus der Gewindestab - bei gleichzeitigem Abstützen des Instrumentes auf der Platte bis auf einen Wert, der 10 % unter der Reißfestigkeit liegt, angezogen.

Diese Position wird auf die folgende Weise fixiert:

Über eine zweite Rändelschraube der Vorrichtung wird die Mutter fingerfest heruntergeschraubt, sodaß sich die Mutter auf der Platte abstützt und die durch die Vorrichtung bisher aufrecht erhaltene axiale Schraubenzugkraft übernimmt. Auf diese Weise kommt es nicht zu einer Drehmomentbelastung des Gewindestabs, da die axiale Schraubenzugkraft durch den reinen Zug an dem in der Vorrichtung eingeschraubten Schraubenende aufgebracht wird, während die Mutter nur soweit heruntergedreht werden muß, bis sie diesen axial aufgebauten Schraubenzug auffängt und aufrecht erhält.

Anschließend wird die Vorrichtung entspannt und der überstehende Gewindestab vermittels eines heißen Drahtes über der Mutter abgeschnitten. Hierbei kann der heiße Draht zugleich quer über den Gewindestab und der Mutter geführt werden, um dabei ein Verschweißen zu erzielen, welches ein Lockerwerden der Verschraubung verhindert.

Weiterhin ist eine kunststoffgerechte Fixierungen des eingedrehten Gewindestabes, z.B. durch einen überstehenden Zapfen möglich, welcher rund oder eckig gearbeitet sein kann und an der Innenseite nach unten hin (d.h. zur Platte bzw. zum Knochen) gerichtete feine Zähne aufweist. Hierüber wird eine passende mit ebensolchen jedoch in entgegengesetzter Richtung angeordneten Zähnen ausgestattete Hülse geschoben, welche durch Anwendung einer nach unten gerichteten Zugkraft auf die Platte geschoben wird und durch das Einrasten der Zähne ineinander eine mechanisch feste, kunststoffgerechte Verbindung herstellt, welche die axiale Schraubenzugkraft weiterhin aufrecht erhält. Diese Hülse ist nach Art einer sich zusammenziehenden elastischen Feder auszugestalten, um beim Herunterschieben der Hülse spontan in den Zähnen einzurasten.

b) Die Gewindestange enthält einen besonders ausgestalteten Ansatz zum Ansetzen eines Werkzeuges bzw. einer Vorrichtung zum Eindrehen der Gewindestange.

In einer weiteren Ausführungsform kann die Vorrichtung zum Befestigen der Osteosyntheseplatte aus einem Gewindestab (3.0) (Fig. 3) und einer Mutter (4.0) (Fig. 4) bestehen, wobei die aus einem resorbierbaren Polymer hergestellte Gewindestange an ihrem oberen Ende mit einem Ansatz - wie z.B. ein Sechskant, Vierkant, Imbus oder einem Querloch (31) - versehen ist (Fig. 3), welches das Eindrehen der Gewindestange in den Knochen ermöglicht. In einer bevorzugten Ausführungsform ist die Gewindestange an dem gegenüberliegenden Ende des für die Drehmomentübertragung gestalteten Kopfes des Gewindes (an der Spitze) (Fig. 3) mit einem oder mehreren Röntgenschatten erzeugenden Teilchen (3.2) versehen, welches selbst aus einem resorbierbaren oder aber auch aus einem nicht-resorbierbaren Material besteht. Die bzw. das den Röntgenschatten erzeugenden Teilchen kann/können jedoch auch auf andere Art und Weise - wie nachfolgend beschrieben - in der Schraube bzw. dem Gewindestab angeordnet sein. Der Gewindestab selbst ist mit einer Sollbruchstelle (3.3) versehen, die bei einer Beschädigung bzw. bei einem vorzeitigen Abbrechen ein leichtes Entfernen des noch im Gewinde befindlichen Restes des Gewindestabes ermöglicht.

Die Gewindestange wird mittels eines durch das Querloch gesteckten Stabes in den Knochen eingeschraubt. Über ein Hilfswerkzeug, welches in das Gewinde der Gewindestange eingreift und welches sich auf der Platte (5.0) (Fig. 5) abstützt, wird der Gewindestab vorgespannt und anschließend über das Gegenstück (4.0) gesichert.

Aus einer derartigen Teilung der bislang aus dem Stand der Technik verwandten Schrauben in eine Art Gewindestange und eine Art Mutter resultieren erhebliche Vorteile:

Durch die Verwendung von Gewindestangen kann die sonst bei Schrauben maximal erreichbare Vorspannkraft nahezu verdoppelt werden.

Die Gewindestange kann in das im Knochen vorbereitete Loch so tief eingeschraubt werden, bis auf dem Röntgenschirm aufgrund des/der im Stab eingebrachten, einen Röntgenkontrast hervorrufenden Teilchen(s) sichergestellt ist, daß die Gewindestange tief genug eingeschraubt ist. Nach dem Aufschrauben des Gegenstückes (4.0) mit Innengewinde wird die Gewindestange mittels eines Hilfsgerätes, welches z.B. in die zur Übertragung eines Drehmomentes vorgesehene Vorrichtungen (3.1) (Fig. 3) eingreift und auf die gewünschte Anzugskraft vorgespannt und das Gegenstück (4.0) (Figs. 4 und 5) festgezogen, was jedoch nicht die Anwendung eines hohen Drehmomentes erfordert, da lediglich die im Gewinde auftretenden Reibungskräfte überwunden werden müssen, nicht aber die Vorspannung über das Anzugsmoment aufgebracht werden muß, wodurch sich das in der Schraube beim Anziehen sonst vorliegende Torsionsmoment erheblich verringert, und letztendlich im wesentlichen nur noch Zugkräfte in der Gewindestange vorliegen. Die erreichbaren Anzugskräfte können in der Befestigungseinheit somit größere Werte annehmen.

Nachdem die Platte befestigt ist, können die überstehenden Enden der verschiedenen Stäbe abgeschnitten werden, wobei anfallende Späne oder Bruchstücke, die unter Umständen in die Wunde gelangen, nicht - wie es z.B. bei Metallspänen der Fall ist -entfernt werden müssen, da sie keine Gewebeirritationen hervorrufen und vom Körper im Laufe der Zeit absorbiert werden. Die überstehenden Stäbe können aber auch - wie schon beschrieben - mittels eines heißen Drahtes entfernt werden, wobei keine Späne anfallen und womit gleichzeitig ein Verschweißen erreicht wird, welches ein Lockerwerden der Verschraubung verhindert.

Bei der Verwendung von faserverstärkten Materialien können aufgrund der quasi einachsigen Kräftewirkung die Fasern axial in die Gewindestäbe eingebracht werden, was gegenüber den auf Torsion und Zug belasteten Schrauben eine wesentliche Verbesserung der Festigkeit sowie eine Vereinfachung der Fertigung bewirkt.

Derartige - beispielsweise im Spritzgrußverfahren hergestellten - Schrauben zeigen aufgrund der in axialer Richtung eingebrachten Orientierung ebenfalls verbesserte Festigkeitswerte.

Da bei Osteosynthesematerialien aus resorbierbaren Polymeren - im Gegensatz zu den entsprechenden Osteosynthesevorrichtungen aus Metall - nicht der Knochen das schwächste Glied ist, sondern die Befestigungseinheit, wird beim Überspannen der Befestigungseinheit die Schraube selbst und nicht der Knochen geschädigt. Da bei der erfindungsgemäßen Lösung, das Stangenende deutlich aus der Platte herausragt, kann durch eine an der Gewindestange angebrachte Verjüngung (3.3) (Fig. 3) eine Sollbruchstelle erzeugt werden, so daß der Stab bei einer eventuellen Überbelastung an dieser vorbestimmten Stelle bricht. Diese gebrochene Gewindestange kann -ohne weitere Manipulationen an der Platte oder gar deren Entfernung, was im anderen Fall bei einer nachfolgend eventuell erforderlichen Verwendung einer Schraube mit größerem Gewindedurchmesser zwangsläufig mit einem Herausschrauben aller bereits angebrachten Schrauben verbunden wäre - ohne weitere Schwierigkeiten von Hand herausgeschraubt werden.

Eine bevorzugte Ausführungsform der erfindungsgemäßen Befestigungseinheit ist in den Figs. 3, 4, 5 und 6 dargestellt und wird im folgenden erläuternd beschrieben:

Die Gewindestange (3.0) (Fig. 3) weist am zur Übertragung des Drehmomentes vorgesehenen Ende ein Querloch (3.1) zur Aufnahme eines Stabes auf, in welches dieser eingestecke oder eingeschraubt werden kann. Am entgegengesetzten Ende - der Spitze - ist ein Röntgenschatten erzeugendes Teilchen (3.2) eingelassen. Die eingebrachte Verjüngung (3.3) ist in diesem Beispiel als Nut gestaltet, kann jedoch auch in jeder anderen geeigneten Ausführungsform gestaltet sein.

Das Gegenstück (4.0) (Fig. 4) ist auf einer Seite als Halbkugel gestaltet, um auch das Anbringen von nicht senkrecht zur Platte stehenden Schrauben zu ermöglichen. Auf der gegenüberliegenden Seite weist das Gegenstück (4.0) (Fig. 4) vier Bohrungungen auf (4.1), die das Verschrauben mit einem entsprechend geeigneten Hilfswerkzeug ermöglichen sollen.

Die Gewindestange wird mittels eines durch das Querloch (3.1) gesteckten Stabes soweit in den Knochen eingeschraubt, bis auf röntgenologischem Wege - die richtige Position der Schraube (Fig. 5) festgestellt wird. Über ein Hilfswerkzeug, welches in das Gewinde des Stabes eingreift und sich auf der Platte (5.0) abstützt wird die Gewindestange vorgespannt und anschließend über das Gegenstück (4.0) gesichert.

Abschließend werden die überstehenden Enden der Gewindestange abgeschnitten.

Das Bohren der Löcher in den Knochenfragmenten kann unter Verwendung einer Bohrschablone oder Bohrlehre, welche auf die Löcher der Osteosyntheseplatte aufgesetzt wird, erfolgen.

Ein weiterer Gegenstand der Erfindung ist - wie schon erwähnt - die Herstellung von Schrauben bzw. Gewindestangen, die röntgenologisch sichtbar gemacht werden können.

Die Schrauben bzw. Gewindestangen erhalten durch heißes Einpressen an der Spitze eine kleine Calciumphosphatkugel, wodurch eine röntgenologische Sichtbarmachung ermöglicht wird. Diese erlaubt die Längenbestimmung der Schraube im Röntgenbild und gibt dem Operateur somit eine Kontrollmöglichkeit an die Hand, zu beurteilen, ob eine Schraube aufgrund fehlerhafter Längenmessung bei der Operation zu lang ist.

Ein Festigkeitsverlust ist hierbei nicht zu befürchten, da lediglich die Spitze der Schrauben leicht erhitzt wird.

Eine weitere Möglichkeit besteht in der Anordnung von mehreren Calciumphosphatgranulat-Kügelchen von der Spitze ab in einem Abstand von jeweils 2 - 3 mm Eine derartig ausgestaltete Schraube ermöglicht es auch nach deren Kürzung in einem direkt postoperativ angefertigten Röntgenbild die Lage der Schraube zu beurteilen bzw. deren Länge zu bestimmen. In einer anderen Ausführungsform wird ein Stift aus Calciumphosphat (mit einem Durchmesser von bis zu ca. 1 mm) mit einer Länge von ca. 5 mm in das Zentrum der Schraube eingebracht, womit ebenfalls die Möglichkeit eröffnet wird, auch nach einer ggf. erforderlichen Kürzung der Schraube deren Lage und Länge auf röntgenologischem Wege zu bestimmen.

In einer weiteren Ausführungsform wird an der distalen Hälfte der Schraubenoberfläche ein sehr feinkörniges Calciumphosphat-Granulat (Durchmesser der Granulatkörner bis max. 300 μm) in der äußersten Schicht im Bereich der Gewindezüge der Schraube aufgebracht, womit diese im Röntgenbild ebenfalls sichtbar gemacht werden kann.

Das Granulat soll lediglich im distalen Bereich der Schraube aufgebracht werden, um eine zu starke Schwächung der Schraube zu verhindern. Neben dem Vorteil, daß auch nach einer - im Zuge der Operation eventuell erforderlichen - Kürzung deren geometrische Form sowie deren Lage auf röntgenologischem Wege feststellbar ist, weist diese Ausführungsform den Vorzug auf, daß durch die Beschichtung der Schraubenoberfläche im distalen Gewindebereich ein Festwachsen der Schraube an der entsprechenden Knochenoberfläche ermöglicht wird, was eine durch die Mikrobewegungen des Körpers bedingte Lockerung oder gar ein eigenständiges Herausdrehen der Schraube erschwert.

## Ansprüche

1) Resorbierbare Vorrichtung zur Osteosynthese bestehend aus einer Osteosyntheseplatte, wobei die dem Knochen zugewandten Seite der Osteosyntheseplatte mit Calciumphosphat und/oder Hydroxylapatit in Form kleiner Teilchen beschichtet ist, und einer Befestigungsvorrichtung, bestehend aus einer Gewindestange, die gegebenenfalls mit Calciumphosphat und/oder Hydroxylapatit in Form kleiner Teilchen beschichtet ist und einer darauf aufsetzbaren Mutter.

2) Resorbierbare Vorrichtung zur Osteosynthese bestehend aus einer Osteosyntheseplatte, dadurch gekennzeichnet, daß die dem Knochen zugewandte Seite mit Calciumphosphat und/oder Hydroxylapatit in Form kleiner Teilchen beschichtet ist. beschichtet ist.

3) Resorbierbare Vorrichtung zum Befestigen einer Osteosyntheseplatte, dadurch gekennzeichnet, daß sie aus einem Gewindestab und einer dazugehörigen Mutter besteht.

4) Resorbierbare Vorrichtung zur Osteosynthese nach Anspruch 1, 2 oder 3, dadurch gekennzeichnet, daß das resorbierbare Material ein Polymerisat oder Copolymerisat auf der Basis von Poly-L-lactid, Poly-D,L-lactid und/oder Polyglykolid ist.

5) Resorbierbare Vorrichtung nach Anspruch 1, 2, 3 oder 4, dadurch gekennzeichnet, daß sie verstärkende Fasern aufweist.

6) Resorbierbare Vorrichtung nach Anspruch 1, 2, 4 oder 5, dadurch gekennzeichnet, daß die Osteosyntheseplatte mit kolloidal verteiltem Silber beschichtet ist.

7) Resorbierbare Vorrichtung nach Anspruch 1, 2, 4, 5 oder 6, dadurch gekennzeichnet, daß die Osteosyntheseplatte mit einem pharmazeutisch wirksamen Material - vorzugsweise einem bakteriostatischen oder bakteriziden Mittel beschichtet ist.

8) Resorbierbare Vorrichtung nach Anspruch 1, 2 oder 4 bis 7, dadurch gekennzeichnet, daß die Summe der Querschnittsflächen, die sich bei einem Schnitt längs der Schnittlinie ff im Bereich der Mitte der Schraubenlöcher ergeben, gleich der Fläche des Querschnitts ist, der sich bei einem Schnitt längs der Schnittlinie ee in dem Bereich zwischen zwei Schraubenlöchern der Osteosyntheseplatte ergibt

9) Resorbierbare Vorrichtung nach Anspruch 1, 2 oder 4 bis 8, dadurch gekennzeichnet, daß die Osteosyntheseplatte in den äußeren Bereichen der Schraubenlöcher entlang der Längsachse Verbreiterungen (7.3) aufweist.

10) Resorbierbare Vorrichtung nach Anspruch 1, 2 oder 4 bis 9, dadurch gekennzeichnet daß die Osteosyntheseplatte in ihrem mittleren Bereich Verstärkungen (7.1) in Form von Verdickungen aufweist, wobei die Verdickungen zu den Enden der Osteosyntheseplatte hin in ihrer Stärke abnehmen.

11) Resorbierbare Vorrichtung nach Anspruch 1, 2 oder 4 bis 10, dadurch gekennzeichnet, daß die Osteosyntheseplatte im Bereich der dem Knochen zugewandten Seite eine Querwölbung aufweist.

12) Resorbierbare Vorrichtung nach Anspruch 1, 3, 4 oder 5, dadurch gekennzeichnet, daß die Gewindestange eine Vorrichtung zum Einschrauben aufweist.

13) Resorbierbare Vorrichtung nach Anspruch 1, 3 bis 5 oder 12, dadurch gekennzeichnet, daß die Gewindestange ein einen Röntgenkontrast erzeugendes Material aufweist.

14) Resorbierbare Vorrichtung nach Anspruch 1, 3 bis 5, 12 oder 13, dadurch gekennzeichnet, daß die Gewindestange eine Sollbruchstelle aufweist.

15) Verfahren zur Herstellung einer resorbierbaren Vorrichtung zur Osteosynthese gemäß einem der Ansprüche 1, 2, 4 bis 11, dadurch gekennzeichnet, daß das Calciumphosphat und/oder Hydroxylapatit-Granulat in Form kleiner Teilchen in die durch Erwärmung erweichte Platte eingepresst wird.

16) Verfahren zur Herstellung einer resorbierbaren Vorrichtung zur Osteosynthese gemäß einem der Ansprüche 1, 2, 4 bis 11, dadurch gekennzeichnet, daß eine Folie aus resorbierbarem. Material auf eine erwärmte Schicht von Calciumphosphat- und/oder Hydroxylapatit-Teilchen aufgepresst wird und die Osteosyntheseplatte durch Beschichten der nicht mit Granulat beschichteten Seite der Folie mit einem resorbierbaren Polymer hergestellt wird.

17) Verfahen zur Herstellung einer resorbierbaren Vorrichtung zur Osteosynthese gemäß einem der Ansprüche 1, 2, 4 bis 11, dadurch gekennzeichnet, daß eine Schicht aus Calciumphosphat- und/oder Hydroxylapatit-Teilchen mit einem resorbierbaren Polymer überspritzt wird.

18) Verwendung einer resorbierbaren Vorrichtung nach einem der Ansprüche 1 bis 13 zur Osteosynthese.

FIG. 1a

FIG. 1b

FIG. 1c

FIG. 2

FIG. 3

3.1

3.3

3.0

3.2

FIG. 4

4.1          4.1

4.0

FIG. 5

3.0

4.0     5.0

FIG. 6

FIG.7a

FIG.7b

7.1

7.2

e —— e

f —— f

7.3

7.0

7.1

7.1

7.3

7.2

FIG.7c

7.1

FIG.7d

7.2    7.1    7.0    7.2

## FIG. 8a

## FIG. 8b

## FIG. 8c

## FIG. 8d

## FIG. 8e